# EUROPÄISCHE PATENTSCHRIFT

(11) **EP 0 673 378 B1**
(45) Veröffentlichungstag und Bekanntmachung des Hinweises auf die Patenterteilung: **12.03.1997**
(21) Anmeldenummer: 94901898.0
(22) Anmeldetag: 27.11.1993
(51) Int. Cl.: C07D 487/04, C07D 495/14, A61K 31/505, C07D 491/147, C07D 471/14

(54) **NEUE TRIAZOLOPYRIMIDONE, IHRE HERSTELLUNG UND VERWENDUNG**
NEW TRIAZOLOPYRIMIDONES, THEIR PREPARATION AND THEIR USE
NOUVELLES TRIAZOLOPYRIMIDONES, LEUR FABRICATION ET LEUR UTILISATION

(30) Priorität: 10.12.1992 DE 4241562
(43) Veröffentlichungstag der Anmeldung: 27.09.1995
(73) Patentinhaber: BASF Aktiengesellschaft, 67063 Ludwigshafen (DE)
(72) Erfinder: SCHLECKER, Rainer, D-67281 Bissersheim (DE); TREIBER, Hans-Joerg, D-68782 Bruehl (DE); BEHL, Berthold, D-67063 Ludwigshafen (DE); HOFMANN, Hans, Peter, D-67117 Limburgerhof (DE)
(86) Internationale Anmeldenummer: EP9303332
(87) Internationale Veröffentlichungsnummer: WO9413673

(56) Entgegenhaltungen:
- EP-A- 0 217 748

## Beschreibung

Die vorliegende Erfindung betrifft neue Triazolopyrimidone, Verfahren zur deren Herstellung sowie deren Verwendung zur Bekämpfung von Krankheiten.

Es sind bereits Pyrazolo- und Triazolochinazoline bekannt, die antiallergische und entzündungshemmende Eigenschaften besitzen (EP 80 176, US 4,053,600, US 4,128,644). Weiter sind Pyrazolochinazoline bekannt, die sich darüber hinaus zur Behandlung von Thrombose und neurologischen Störungen eignen (US 5,153,196).

Es wurde nun gefunden, daß Triazolopyrimidone der Formel I 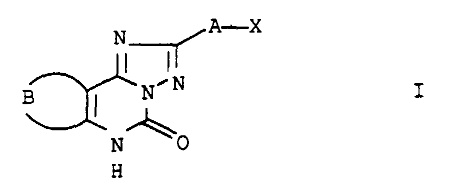 worin
- A: eine direkte Bindung oder eine C₁₋₃-Alkylenkette,
- B: eine C₃₋₆-Alkylenkette, die durch ein Stickstoff-, Schwefeloder Sauerstoffatom unterbrochen sein kann,
- X: eine Carboxylgruppe, die in Form ihres Salzes mit einem physiologisch verträglichen Amin- oder Metallkation vorliegen kann; den Rest 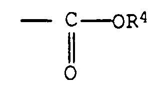 wobei R⁴ einen C₁₋₈-Alkylrest, eine Cycloalkylgruppe mit 3 bis 8 Kohlenstoffatomen im Ring, einen Benzylrest, einen der Reste -(CH₂)ₙ-O-R⁵ oder 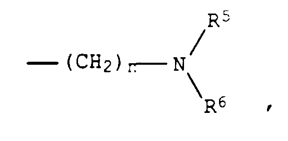 in denen n die Zahl 2, 3 oder 4 und R⁵ und R⁶ für eine C₁₋₃-Alkylgruppe stehen; eine Hydroxy-C₁₋₄-alkyl-, Nitrilo-C₁₋₄-alkyl-, Tetrazolyl-, Carbonylaminotetrazol-, C₁₋₄-Alkylcarbonyl- oder eine gegebenenfalls substituierte Carbamoylgruppe
bedeuten, ein anderes Wirkungsspektrum zeigen.

Als Substituenten A-X seien beispielsweise die Reste folgender Verbindungen genannt:
Ameisensäure, Essigsäure, 2-Propionsäure, 3-Propionsäure, 4-Buttersäure, 3-Buttersäure, 2-Buttersäure, 5-Valeriansäure, 4-Valeriansäure, 3-Valeriansäure, 2-Valeriansäure sowie ihre jeweiligen Methyl-, Ethyl-, Propyl-, Isopropyl-, Butyl-, Pentyl-, Hexyl-, Heptyl-, Cyclopropyl-, Cyclobutyl-, Cyclopentyl-, Cyclohexyl-, Cycloheptyl- und Cyclooctylester oder ihre Amide, wie die Methylamide, Dimethylamide, Ethylamide, Diethylamide, Propylamide, Butylamide, Benzylamide; Hydroxymethan, 1-Hydroxyethan, 2-Hydroxyethan, 1-Hydroxypropan, 2-Hydroxypropan, 3-Hydroxypropan, 1-Hydroxybutan, 2-Hydroxybutan, 3-Hydroxybutan, 4-Hydroxybutan, Hydroxypentan, Hydroxyheptan; Methoxymethan, Methoxyethan, Methoxypropan, Methoxybutan, Ethoxymethan, Ethoxypropan, Ethoxybutan. Oxomethan, 1-Oxoethan, 2-Oxoethan, 1-Oxopropan, 2-Oxopropan, 3-Oxopropan, 1-Oxobutan, 2-Oxobutan, 3-Oxobutan, 4-Oxobutan, 1-Oxopentan, 2-Oxopentan, 3-Oxopentan, 4-Oxopentan; Cyanomethan, Cyanoethan, 1-Cyanopropan, 2-Cyanopropan, 3-Cyanopropan, 1-Cyanobutan, 2-Cyanobutan, 3-Cyanobutan, 4-Cyanobutan.

Für B seien folgende Reste speziell aufgeführt:

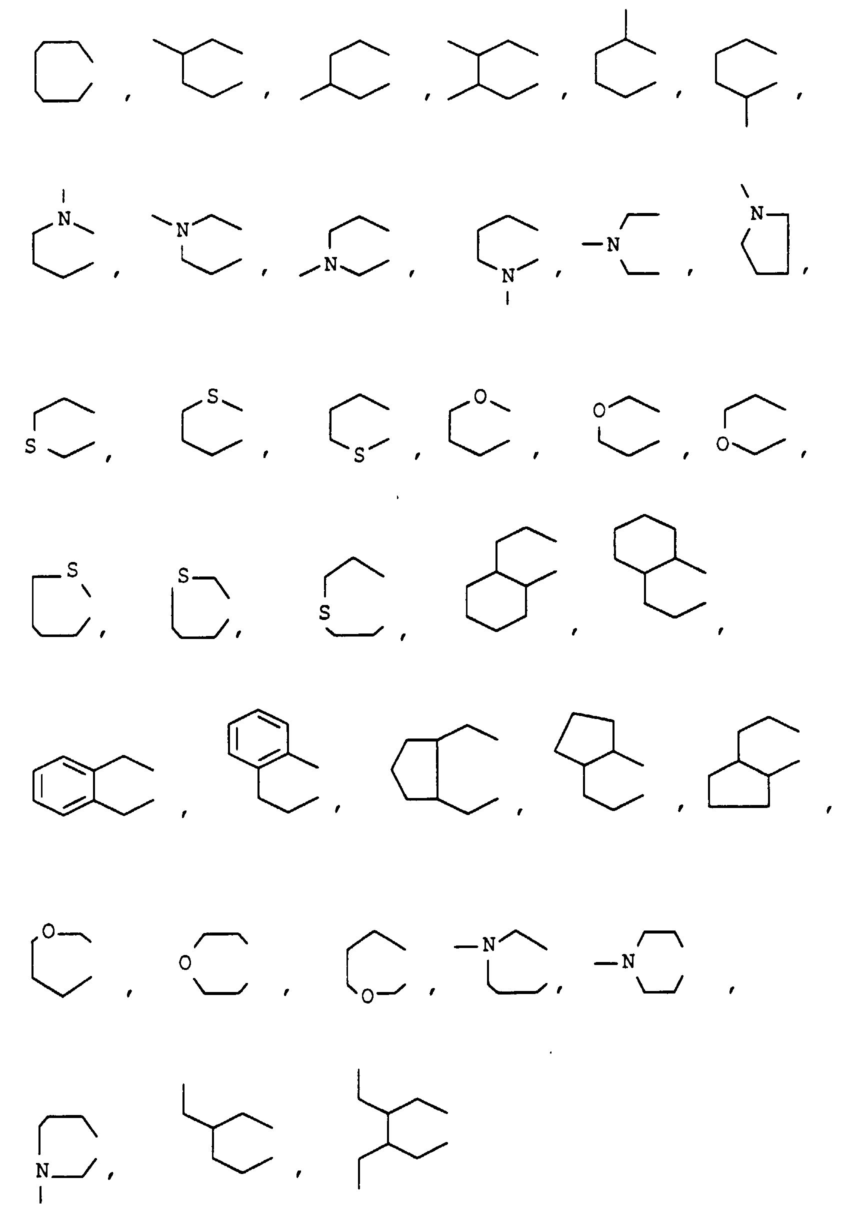

Die Herstellung der Verbindungen der Formel I erfolgt durch eine intramolekulare Kondensationsreaktion eines Hydrazinopyrimidins der Formel II 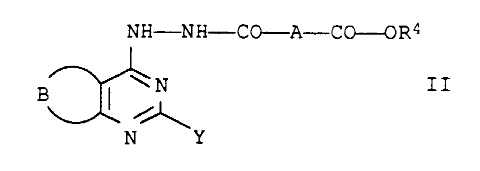 in der A, B, X und R⁴ die für die Formel I angegebene Bedeutung haben und Y eine Hydroxylgruppe oder ein Brom- oder Chloratom darstellt, vorzugsweise in Anwesenheit eines Dehydratisierungsmittels, insbesondere von Phosphoroxychlorid, Polyphosphorsäure oder Essigsäure, gegebenenfalls in einem inerten Lösungsmittel, wie Toluol, Chlorbenzol, Xylol oder überschüssiger Essigsäure, bei Temperaturen von 50 bis 150°C, und zwar vorzugsweise bei der Rückflußtemperatur der Reaktionsmischung.

Die so erhaltenen Ester können anschließend verseift und die freien Säuren mit einem Amin oder einem Metallkation in physiologisch verträgliche Salze überführt werden. Die freien Säuren können auch in die Hydroxyalkylverbindungen (X = Hydroxyalkyl) reduziert oder nach bekannten Methoden in die Nitrile, Tetrazolamino- und Carbamoylverbindungen überführt werden.

Die Herstellung von Verbindungen der Formel I, in denen X eine Carboxylgruppe darstellt, erfolgt durch Hydrolyse der entsprechenden Ester vorzugsweise unter alkalischen Bedingungen, beispielsweise in Gegenwart eines Alkalimetallhydroxids oder Natriumhydrogencarbonats, in einem Lösungsmittel, wie Wasser, einem niederen Alkohol, Tetrahydrofuran oder Mischungen derselben. Die so erhaltenen organischen Säuren werden gegebenenfalls in ein physiologisch verträgliches Amin- oder Metallsalz überführt. Darunter versteht man insbesondere Salze der Alkalimetalle, wie Natrium und Kalium, der Erdalkalimetalle, wie Calcium, sonstiger Metalle, wie Aluminium, sowie Salze von organischen Basen, wie Morpholin, Piperidin, Mono-, Di- und Triethanolamin oder Tris(hydroxymethyl)aminomethan, die dem Fachmann allgemein bekannt sind.

Carbonsäuren der Formel I können weiterhin hergestellt werden durch Hydrogenolyse der entsprechenden Benzylester nach bekannten Methoden, wie sie beispielsweise in Houben-Weyl Bd. IV/lc S. 381ff beschrieben sind. Die Reaktion erfolgt in Anwesenheit eines Katalysators, wie Platin, Palladium oder Nickel, zweckmäßig auf einem Träger, insbesondere Kohle, in einem Lösungsmittel, wie einem niederen Alkohol, insbesondere Methanol, Essigsäure oder einem Dialkylformamid, insbesondere Dimethylformamid, bei Temperaturen von 0°C bis zum Siedepunkt des Lösungsmittels, und vorzugsweise unter nur leicht erhöhtem Druck.

Amide der Formel I, in der X für eine Carbamoylgruppe steht, werden durch Umsetzung der Ester mit Ammoniak oder Amin in Gegenwart eines Lösungsmittels wie Wasser, eines niederen Alkohols, einer wäßrig-alkoholischen Lösung oder Dialkylformamid bei Temperaturen zwischen 0°C und der Rückflußtemperatur des Systems erhalten.

Durch Behandlung primärer Amide mit einem Dehydratisierungsmittel, wie Phosphorpentoxid, Phosphoroxychlorid oder Thionylchlorid, erhält man die Nitrile der Verbindungen der Formel I, worin X eine Nitrilgruppe bedeutet. Die Reaktion wird im allgemeinen mit einem Überschuß des Dehydratisierungsmittels bei der Rückflußtemperatur der Mischung durchgeführt. Gegebenenfalls kann die Reaktion in Gegenwart eines inerten Lösungsmittels, wie Benzol oder Ethylenchlorid, durchgeführt werden.

Die Synthese von Verbindungen der Formel I, in denen X für einen Tetrazolrest steht, erfolgt nach an sich bekannten Methoden, wie sie beispielsweise in Synth. 1973, 80 beschrieben sind, durch Umsetzung der Amide mit Stickstoffwasserstoffsäure oder einem ihrer Salze, beispielsweise mit Alkali- oder Erdalkaliaziden, gegebenenfalls in Anwesenheit von Lewissäuren wie Aluminium- und Zinnchlorid oder von Ammoniumchlorid. Bevorzugt ist die Kombination von Natriumazid mit Ammoniumchlorid. Im allgemeinen wird die Reaktion in Anwesenheit eines inerten Lösungsmittels wie Benzol, Tetrahydrofuran oder Dimethylformamid bei Temperaturen zwischen Raumtemperatur und 150°C durchgeführt. Die Tetrazolylverbindungen sind stark sauer und können in üblicher Weise in ein Salz mit einem physiologisch verträglichen Amin- oder Metallkation überführt werden.

Die Reduktion von Carbonsäuren, insbesondere eines Esters einer Verbindung der Formel I, nach bekannten Verfahren, beispielsweise mit Hilfe eines komplexen Metallhydrids, wie Lithiumborhydrid, in Gegenwart eines Ethers, wie Tetrahydrofuran als Lösungsmittel liefert die Hydroxymethylverbindungen der Formel I (X = CH₂OH). Die Reduktion wird vorzugsweise beim Siedepunkt der Reaktionsmischung durchgeführt.

Verbindungen der Formel I mit einem Carbonylaminotetrazolrest für X (X = CO-NH-CHN₄) können nach bekannten Methoden durch Kondensation der zugrundeliegenden Carbonsäure mit 5-Aminotetrazol der Formel III 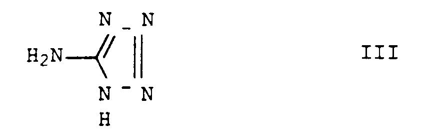 erhalten werden. Die Reaktion wird in der Regel in einem inerten Lösungsmittel, wie beispielsweise Methylenchlorid, Dioxan, Tetrahydrofuran oder Dimethylformamid, bevorzugt in Anwesenheit eines aus der Peptidchemie bekannten Kondensationsreagenzes, wie N,N'-Carbonyldiimidazol oder N,N'-Dicyclohexylcarbodiimid, bei Temperaturen von 20°C bis 120°C durchgeführt.

In analoger Weise können auch Verbindungen der Formel I, in der X für einen gegebenenfalls substituierten Carbamoylrest steht, aus den entsprechenden Säuren hergestellt werden.

Die Herstellung der Ausgangsverbindungen der Formel II erfolgt in an sich bekannter Weise durch Kondensation eines Hydrazinopyridons der Formel IV mit einem Acylhalogenid der

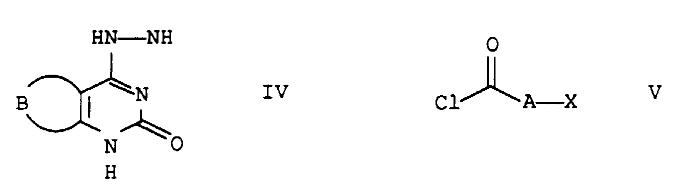

Formel V oder einem entsprechenden Ester.

Bei Verwendung eines Acylhalogenides, bevorzugt eines Chlorids, erfolgt die Reaktion zweckmäßig bei Temperaturen von -30°C bis 70°C, vorzugsweise bei Raumtemperatur, in einem inerten Lösungsmittel wie Dimethylformamid, Dioxan, Tetrahydrofuran oder Methylenchlorid. Die Umsetzung wird bevorzugt in Anwesenheit von tertiären organischen Basen, wie Triethylamin oder Pydridin, durchgeführt.

Die Umsetzung von IV mit Estern kann mit oder ohne Lösungsmittel wie Toluol, Chlorbenzol oder Diphenylether, bei einer Temperatur von etwa 20°C bis zur Rückflußtemperatur der Mischung durchgeführt werden. Die Ester der Formel I können nach bekannten Verfahren, wie sie beispielsweise im Houben-Weyl, Bd. 8, S. 526 bis 528 beschrieben sind, mit Alkoholen zu Estern mit einem anderen Rest R⁴ umgeestert werden.

Verbindungen der Formel I, in denen X für Nitril, Tetrazol oder Hydroxymethyl steht und direkt an den Heterocyclus gebunden ist, werden bevorzugt nach oben beschriebenen Verfahren aus der entsprechenden Säure synthetisiert.

Ein weiteres Verfahren zur Herstellung von Ausgangsverbindungen der Formel II besteht in der Reaktion eines Hydrazins der Formel VI mit einem Pyrimidin der Formel VII, worin X für eine nucleofuge Abgangsgruppe steht.

H₂NNHCOAX VI

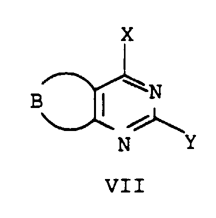

Die Reaktion wird zwischen 0°C und 50°C in einem inerten Lösungsmittel wie Ethanol, Methylenchlorid, Toluol, Tetrahydrofuran oder Dimethylformamid, vorzugsweise mit einem Überschuß an VI, durchgeführt.

Die Verbindungen der Formel IV werden zweckmäßigerweise durch Kondensation von einer Verbindung der Formel VII mit Hydrazin hergestellt. Das Verfahren wird in an sich bekannter Weise durchgeführt, d.h. im allgemeinen bei Temperaturen von -20°C bis 50°C in einem inerten Lösungsmittel wie Dioxan, Tetrahydrofuran, Methylenchlorid oder Dimethylformamid.

Die Verbindungen der Formel VII, in denen X und Y Chlor oder Brom bedeuten, lassen sich nach allgemein bekannten Methoden durch Umsetzung der entsprechenden Pyrimidin-2,4-dione mit Phosphoroxychlorid oder -bromid erhalten. Diese Reaktionen sind ebenso wie die Synthese der Pyrimidindione in The Chemistry of Het. Compounds, The Pyrimidines, Wiley 1962, New York, beschrieben.

Die erfindungsgemäßen Verbindungen eignen sich zur Behandlung von Erkrankungen des Zentralnervensystems. Hierzu zählen insbesondere Erkrankungen, die auf eine akute oder chronische Störung der Blutversorgung und/oder des zellulären Stoffwechsels des Zentralnervensystems zurückzuführen sind, wie beispielsweise ischämische zerebrale Insulte, vaskuläre Enzephalopathien und die daraus resultierenden dementiellen Erkrankungen, Epilepsien, Parkinson-Krankheit, Depressionen, Migräne sowie traumatische Läsionen des Gehirns und des Rückenmarks.

Die pharmakologische Wirksamkeit der erfindungsgemäßen Verbindungen I wurde an isoliertem Membranmaterial von Rattengroßhirnen untersucht. Hierzu wurde das Membranmaterial in Gegenwart der erfindungsgemäßen Verbindungen mit den radioaktiv markierten Substanzen ³H-2-Amino-3-hydroxy-5-methyl-4-isoxazol-propionsäure (³H-AMPA) und ³H-5,7-Dichlorkynurensäure behandelt, wobei sich diese an spezifische Rezeptoren (AMPA- bzw. NMDA-Rezeptor (N-Methyl-D-aspartat)) binden. Anschließend wurde durch Scintillationszählung die Radioaktivität der behandelten Membranen gemessen. Über die gebundene Radioaktivität ließen sich die Mengen an gebundener ³H-AMPA und 3H-5,7-Dichlorkynurensäure bzw. jeweils die verdrängten Mengen dieser radioaktiv markierten Substanzen bestimmen. Die sich hieraus ergebende Dissoziationskonstante K_{I} (I = Inhibitor), welche ein Maß für die Verdrängungswirkung des erfindungsgemäßen Wirkstoffes ist, wurde durch iterative nichtlineare Regressionsanalyse mit dem Statistical Analysis System (SAS) an einem IBM-Rechner, ähnlich dem Programm "Ligand" von P.J. Munson und D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:
1. Bindung von ³H-2-Amino-3-hydroxy-5-methyl-4-isoxazol-propionsäure (³H-AMPA)
Für die Präparation des Membranmaterials wurden frisch entnommene Rattengroßhirne zusammen mit dem ca. 15fachen Volumen einer Pufferlösung A aus 30 mM α,α,α-Tris-(hydroxymethyl)-methylamin-Hydrochlorid (TRIS-HCl) und 0,5 mM Ethylendiamintetraessigsäure (EDTA) - pH 7,4 - mittels eines Ultra-TURRAX-Rührers homogenisiert. Die Suspension wurde 20 Minuten bei 48 000 g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20minütiges Zentrifugieren bei 48 000 g gewaschen. Danach wurde das Membranmaterial in einem 15fachen Volumen der Pufferlösung A suspendiert und 30 Minuten bei 37°C inkubiert. Anschließend wurde das Proteinmaterial zweimal durch Zentrifugieren und Suspendieren gewaschen und bis zur Verwendung bei -70°C eingefroren.
Für den Bindungstest wurde das bei 37°C aufgetaute Proteinmaterial zweimal durch Zentrifugieren bei 48 000 g (20 Minuten) und anschließendes Suspendieren in einer Pufferlösung B aus 50 mM TRIS-HCl, 0,1 M Kaliumthiocyanat und 2,5 mM Calciumchlorid - pH 7,1 - gewaschen. Anschließend wurden 0,25 mg Membranmaterial, 0,1 µCi ³H-AMPA (60 Ci/mmol) sowie Verbindung I in 1 ml Pufferlösung B gelöst und 60 Minuten auf Eis inkubiert. Die inkubierte Lösung wurde über einen CF/B-Filter (Firma Whatman), der zuvor mindestens 2 Stunden mit einer 0,5 %igen wäßrigen Lösung von Polyethylenimin behandelt worden war, filtriert. Anschließend wurde das Filtrat mit 5 ml kalter Pufferlösung B gewaschen, um gebundene und freie ³H-AMPA voneinander zu trennen. Nach Messung der Radioaktivität der gebundenen 3H-AMPA im Membranmaterial durch Scintillationszählung wurde durch Auswertung der Verdrängungskurven mittels Regressionsanalyse der K_{I}-Wert bestimmt.
In diesem Versuch wurden folgende Ergebnisse erhalten:

| Substanz des Beispiels Nr. | AMPA-Bindung Kᵢ [µM] |
|---|---|
| 3 | 1,5 |
| 10 | 1,7 |
| 11 | 1,0 |
| 21 | 1,7 |

2. Bindung von ³H-5,7-Dichlorkynurensäure
Für die Präparation des Membranmaterials wurden frisch entnommene Rattengroßhirne zusammen mit dem ca. 10fachen Volumen einer Pufferlösung A' aus 50 mM TRIS-HCl und 10 mM EDTA - pH 7,4 - homogenisiert. Die Suspension wurde 20 Minuten bei 48 000 g zentrifugiert. Nach Abtrennung der überstehenden Flüssigkeit wurde das im Bodensatz enthaltene Membranmaterial zweimal durch Suspendieren in der Pufferlösng A' und anschließendes jeweils 20minütiges Zentrifugieren und Suspendieren gewaschen. Nach erneutem Suspendieren der Membranen in der Pufferlösung A' und Einfrieren in flüssigem Stickstoff wurde die Suspension wieder bei 37°C aufgetaut und nach einem weiteren Waschvorgang 15 Minuten bei 37°C inkubiert. Anschließend wurde das Proteinmaterial viermal durch Zentrifugieren und Suspendieren gewaschen und bis zur Verwendung bei -70°C eingefroren.
Für den Bindungstest wurde das bei 37°C aufgetaute Proteinmaterial zweimal durch Zentrifugieren bei 48 000 g (20 Minuten) und anschließendes Suspendieren in einer Pufferlösung B' aus 50 mM TRIS-HCl - pH 7,4 - gewaschen. Anschließend wurden 0,15 mg Membranmaterial, 0,3 µCi ³H-5,7-Dichlorkynurensäure (16 Ci/mmol) sowie Verbindung I in 1 ml Pufferlösung B' gelöst und 30 Minuten auf Eis inkubiert. Die inkubierte Lösung wurde 2 Minuten bei 150 000 g zentrifugiert. Nach Abtrennung der überstehenden Flüssigkeit wurden die Bodensätze zweimal mit je 1,5 ml kalter Pufferlösung B' suspendiert. Nach Messung der Radioaktivität der an die Membranen gebundenen ³H-5,7-Dichlorkynurensäure im Bodensatz ergab sich der K_{I}-Wert durch Auswertung der Verdrängungskurven mittels der Regressionsanalyse.
In diesem Versuch wurden folgende Ergebnisse erhalten:

| Substanz des Beispiels Nr. | Bindung von Dichlorkynurensäure Kᵢ [µM] |
|---|---|
| 2 | 0,2 |
| 3 | 1,2 |
| 6 | 1,6 |
| 7 | 0,4 |
| 8 | 0,2 |
| 9 | 0,65 |
| 10 | 0,3 |
| 11 | 0,1 |
| 12 | 1,6 |
| 15 | 2,0 |
| 18 | 0,8 |
| 21 | 2,6 |

Die Herstellung der Arzneimittelzubereitungen erfolgt in üblicher Weise, z.B. durch Vermischen des Wirkstoffes mit den anderen üblichen Trägerstoffen und Verdünnungsmitteln.

Die Arzneimittelzubereitungen können in verschiedenen Applikationsweisen verabreicht werden, wie peroral, parenteral, subkutan, intraperitoneal und topisch. So sind Zubereitungsformen wie Tabletten, Emulsionen, Infusions- und Injektionslösungen, Pasten, Salben, Gele, Cremes, Lotionen, Puder und Sprays möglich.

Die erfindungsgemäßen Arzneimittelzubereitungen enthalten neben den üblichen Arzneimittelhilfsstoffen eine therapeutisch wirksame Menge der Verbindungen I. Für die lokale äußere Anwendung, z.B. in Puder und Salben, können die Wirkstoffe in den üblichen Konzentrationen enthalten sein. In der Regel sind die Wirkstoffe in einer Menge von 0,001 bis 5 Gew.-%, vorzugsweise 0,01 bis 0,5 Gew.-%, enthalten.

Bei der inneren Anwendung werden die Präparationen in Einzeldosen verabreicht. In einer Einzeldosis werden pro kg Körpergewicht 0,1 bis 50 mg, vorzugsweise 0,1 bis 10 mg, Wirkstoff gegeben. Die Zubereitungen können täglich in einer oder mehreren Dosierungen je nach Art und Schwere der Erkrankungen verabreicht werden. Die Tagesdosis liegt in der Regel bei 0,1 bis 100 mg pro kg Körpergewicht bei oraler Gabe und 0,01 bis 10 mg pro Körpergewicht bei parenteraler Gabe.

Entsprechend der gewünschten Applikationsart enthalten die erfindungsgemäßen Arzneimittelzubereitungen neben dem Wirkstoff die üblichen Trägerstoffe und Verdünnungsmittel. Für die lokale äußere Anwendung können pharmazeutisch-technische Hilfsstoffe wie Ethanol, Isopropanol, oxethyliertes Ricinusöl, oxethyliertes hydriertes Ricinusöl, Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Bleichmittel enthalten sein.

Die neben dem Wirkstoff in der Zubereitung enthaltenen Stoffe sowie die bei der Herstellung der pharmazeutischen Zubereitung verwendeten Stoffe müssen toxokologisch unbedenklich und mit dem jeweiligen Wirkstoff verträglich sein.

### Beispiele

I. Synthese einiger Zwischenstufen
   A 2-Chlor-4-hydrazino-7,8,9,10-tetrahydrochinazolin
      113 g 2,4-Dichlor-7,8,9,10-tetrahydrochinazolin wurden in 1 Liter Methylenchlorid gelöst und bei 20°C mit 113 ml Hydrazinhydrat versetzt. Die Lösung wurde über Nacht gerührt, das Lösungsmittel abdestilliert, der Rückstand mit Wasser gerührt, getrocknet und mit Methyl-tert.-butylether behandelt. Ausbeute: 90,2 g.
   B 2-Chlor-4-N(N'-ethyloxalylhydrazino)-7,8,9,10-tetrahydrochinazolin
      1,5 g aus Beispiel A werden in 50 ml CH₂Cl₂ 1,2 ml Triethylamin mit 1,2 g Oxalsäureethylesterchlorid versetzt. Die Mischung wird über Nach bei Raumtemperatur gerührt, das Lösungsmittel abgezogen, der Rückstand mit Wasser gerührt und getrocknet. Ausbeute: 1,8 g.
II. Synthese erfindungsgemäßer Verbindungen
   Beispiel 1
      7,8,9,10-Tetrahydro-1,2,4-triazolo[1,5-c]chinazolin-5-on-2-carbonsäureethylester
      35 g 2-Chlor-4-N-(N'-ethyloxalylhydrazino-7,8,9,10-tetrahydrochinazolin wurden in 250 ml Essigsäure 2,5 h unter Rückfluß erhitzt. Das Lösungsmittel wurde abdestilliert und der Rückstand mit MTB gerührt, abgesaugt und getrocknet. Ausbeute: 25,5 g. Fp. 266 - 268°C.
   Beispiel 2
      7,8,9,10-Tetrahydro-1,2,4-triazolo[l,5-c]chinazolin-5-on-2-carbonsäure
      5 g Ester aus Beispiel 1 wurden in 120 ml 1 n NaOH 4 h bei Raumtemperatur gerührt. Die Lösung wurde mit MTB extrahiert, angesäuert, der Niederschlag abgesaugt, mit Wasser gewaschen und getrocknet. Ausbeute: 3,2 g. Fp. 188°C.
   Beispiel 3
      7,8,9,10-Tetrahydro-1,2,4-triazolo[1,5-c]chinazolin-5-on-2-carbonsäure-N-benzylamid
      1,4 g Säure aus Beispiel 2 wurden in 20 ml Methylenchlorid mit 0,75 g N-Hydroxysuccinimid und 1,25 g Dicyclohexylcarbodiimid versetzt. Die Mischung wurde über Nacht bei Raumtemperatur gerührt, der Feststoff abgesaugt, intensiv mit Methylenchlorid gewaschen und in 50 ml Methylenchlorid resuspendiert. Anschließend wurden 0,63 g Benzylamin zugegeben, über Nacht bei Raumtemperatur gerührt, der Feststoff abgesaugt und mit heißem Ethanol ausgekocht. Ausbeute: 0,9 g. Fp. 255 - 259°C.
      Analog Beispiel 1-3 wurden folgende Verbindungen der Formel I hergestellt:

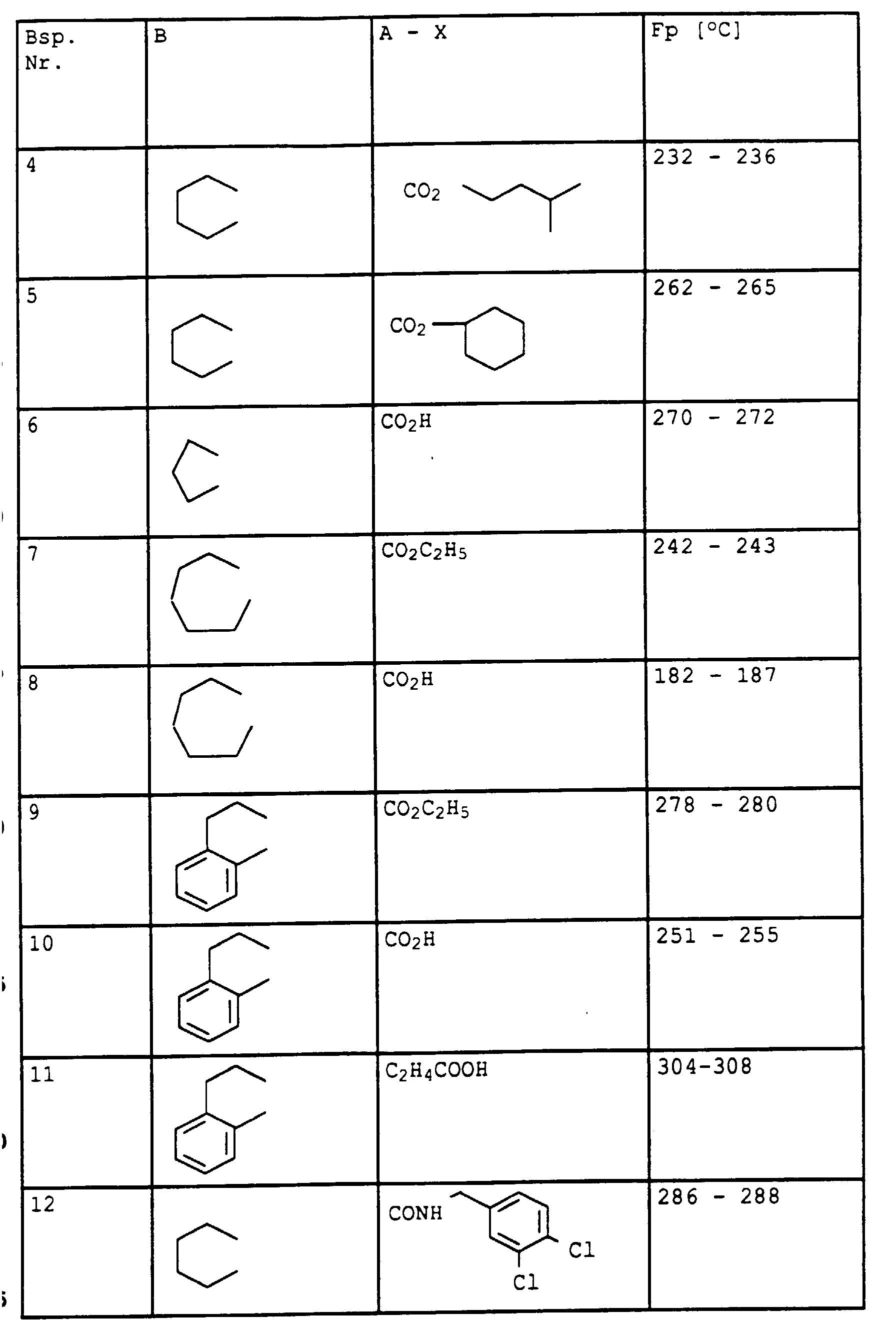

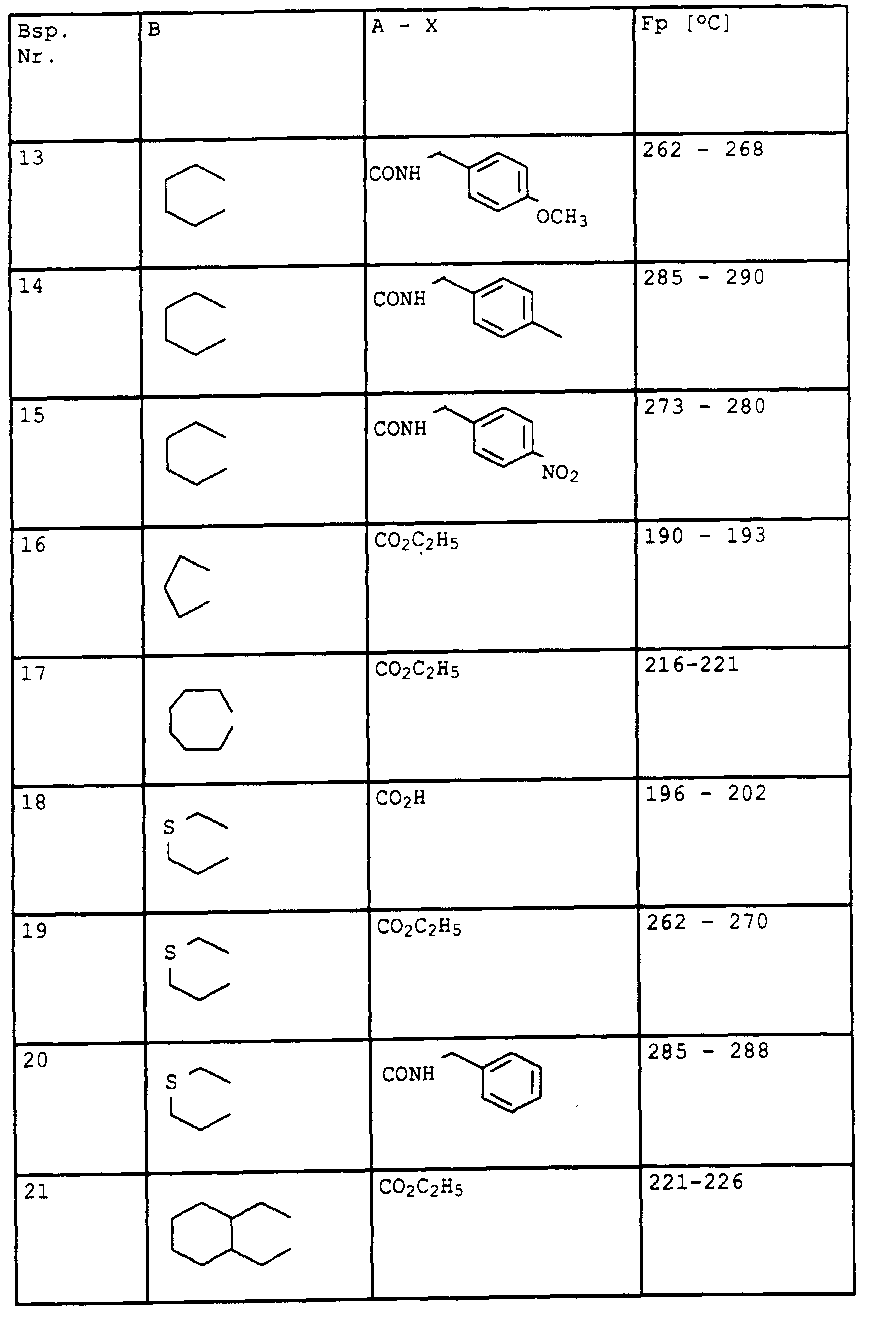

## Patentansprüche

1. Triazolopyrimidone der Formel I 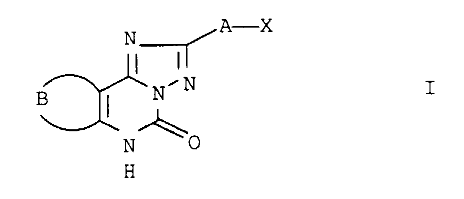 worin
A eine direkte Bindung oder eine C₁₋₃-Alkylenkette,
B eine C₃₋₆-Alkylenkette, die durch ein Stickstoff-, Schwefel- oder Sauerstoffatom unterbrochen sein kann,
X eine Carboxylgruppe, die in Form ihres Salzes mit einem physiologisch verträglichen Amin- oder Metallkation vorliegen kann; den Rest 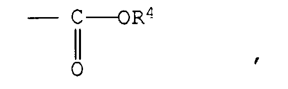 wobei R⁴ einen C₁₋₈-Alkylrest, eine Cycloalkylgruppe mit 3 bis 8 Kohlenstoffatomen im Ring, einen Benzylrest, einen der Reste -(CH₂)ₙ-O-R⁵ oder 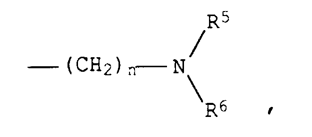 in denen n die Zahl 2, 3 oder 4 und R⁵ und R⁶ für eine C₁₋₃-Alkylgruppe stehen; eine Hydroxy-C₁₋₄-alkyl-, Nitrilo-C₁₋₄-alkyl-, Tetrazolyl-, Carbonylaminotetrazol- oder eine C₁₋₄-Alkylcarbonylgruppe
bedeuten.

2. Verfahren zur Herstellung der Triazolopyrimidone der Formel I gemäß Anspruch 1, dadurch gekennzeichnet, daß man ein Hydrazinopyrimidin der Formel II 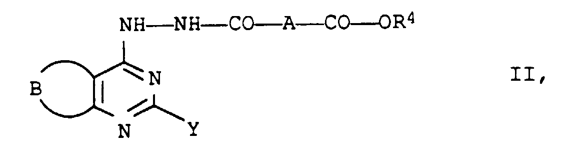 worin A, B, X und R⁴ die angegebene Bedeutung besitzen, intramolekular kondensiert und die so erhaltene Verbindung der Formel I (X=CO-OR⁴) gegebenenfalls anschließend reduziert oder verseift und so erhaltene freie Säuren gegebenenfalls in ihre physiologisch verträglichen Salze überführt oder zu den Alkoholen reduziert oder in die Nitrile, Tetrazolamino- oder Carbamoylverbindungen umwandelt.

3. Triazolopyrimidone der Formel I gemäß Anspruch 1 zur Verwendung bei der Bekämpfung von Krankheiten.

4. 7,8-Benzo-7,8,9,10-tetrahydro-1,2,4-triazolo[1,5-c]chinazolin-5-on-2-carbonsäureethylester

5. 7,8-Benzo-7,8,9,10-tetrahydro-1,2,4-triazolo[1,5-c]chinazolin-5-on-2-carbonsäure

6. 7,8-Benzo-7,8,9,10-tetrahydro-1,2,4-triazolo[1,5-c]chinazolin-5-on-2-propionsäure

7. 8,9-Tetramethylen-7,8,9,10-tetrahydro-1,2,4-triazolo[1,5-c]chinazolin-5-on-2-carbonsäureethylester

## Claims

1. A triazolopyrimidone of the formula I 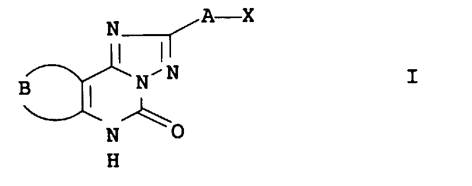 where
A is a direct linkage or a C₁-C₃-alkylene chain,
B is a C₃-C₆-alkylene chain which can be interrupted by a nitrogen, sulfur or oxygen atom,
X is a carboxyl group which may be in the form of its salt with a physiologically tolerated amine or metal cation; the radical 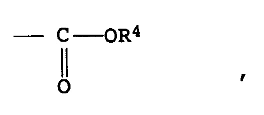 where R⁴ is a C₁₋₈-alkyl radical, a cycloalkyl group with 3 to 8 carbon atoms in the ring, a benzyl radical, one of the radicals -(CH₂)ₙ-O-R⁵ or 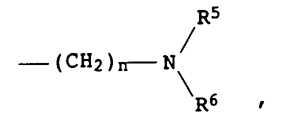 where n is the number 2, 3 or 4 and R⁵ and R⁶ are each a C₁₋₃-alkyl group, a hydroxy-C₁₋₄-alkyl, cyano-C₁₋₄-alkyl, tetrazolyl, tetrazolylcarbamoyl or a C₁₋₄-alkylcarbonyl group.

2. A process for preparing triazolopyrimidones of the formula I as claimed in claim 1, which comprises a hydrazinopyrimidine of the formula II 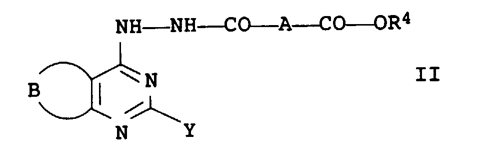 where A, B, X and R⁴ have the stated meanings, being subjected to intramolecular condensation, and the resulting compound of the formula I (X=CO-OR⁴) subsequently being reduced or hydrolyzed where appropriate, and the resulting free acids being where appropriate converted into their physiologically tolerated salts or reduced to the alcohols or converted into the nitriles, tetrazolamino or carbamoyl compounds.

3. A triazolopyrimidone of the formula I as claimed in claim 1 for use for controlling diseases.

4. Ethyl 7,8-benzo-7,8,9,10-tetrahydro-1,2,4-triazolo[1,5-c]quinazolin-5-one-2-carboxylate.

5. 7,8-Benzo-7,8,9,10-tetrahydro-1,2,4-triazolo[1,5-c]quinazolin-5-one-2-carboxylic acid.

6. 7,8-Benzo-7,8,9,10-tetrahydro-1,2,4-triazolo[1,5-c]quinazolin-5-one-2-propionic acid.

7. Ethyl 8,9-tetramethylene-7,8,9,10-tetrahydro-1,2,4-triazolo[1,5-c]quinazolin-5-bne-2-carboxylate.

## Revendications

1. Triazolopyrimidones de la formule I 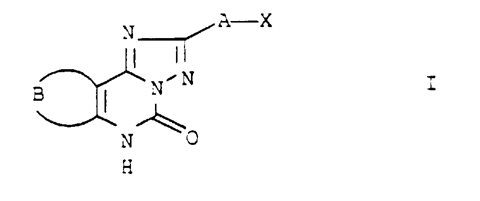 dans laquelle
A représente une liaison directe ou une chaîne alkylène en C₁ à C₃,
B représente une chaîne alkylène en C₃ à C₆, qui peut être interrompue par un atome d'azote, de soufre ou d'oxygène,
X représente un radical carboxyle qui peut se présenter sous forme de son sel avec un cation métal ou amine physiologiquement acceptable; le reste 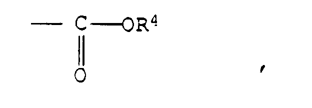 où R⁴ représente un radical alkyle en C₁ à C₈, un radical cycloalkyle comportant de 3 à 8 atomes de carbone dans le cycle, un radical benzyle, un des restes -(CH₂)ₙ-O-R⁵ ou 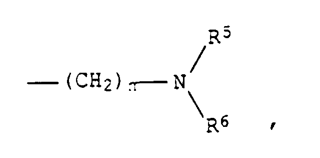 dans lesquels n représente 2, 3 ou 4 et les symboles R⁵ et R⁶ représentent chacun un groupe alkyle en C₁ à C₃; un radical hydroxyalkyle en C₁ à C₄, nitroloalkyle en C₁ à C₄, tétrazolyle, carbonylaminotétrazole ou alkyl(C₁-C₄)carbonyle.

2. Procédé de préparation des triazolopyrimidones de la formule I suivant la revendication 1, caractérisé en ce que l'on condense une hydrazinopyrimidine de la formule II 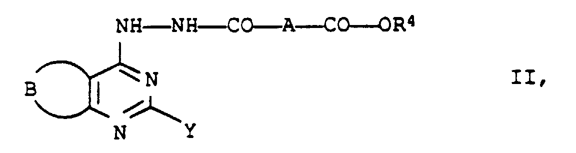 dans laquelle les symboles A, B, X et R⁴ possèdent les significations qui leur ont été précédemment attribuées, de façon intramoléculaire et on réduit ou saponifie ensuite éventuellement le composé de la formule I (X=CO-OR⁴) ainsi obtenu et on convertit éventuellement les acides libres ainsi obtenus en leurs sels physiologiquement acceptables, ou on les réduit en alcools, ou on les transforme en les composés du type nitrile, tétrazolamino ou carbamoyle.

3. Triazolopyrimidones de la formule I suivant la revendication 1, à utiliser pour la lutte contre des maladies.

4. 7,8-Benzo-7,8,9,10-tétrahydro-1,2,4-triazolo[1,5-c]quinazoline-5-one-2-carboxylate d'éthyle

5. Acide 7,8-Benzo-7,8,9,10-tétrahydro-1,2,4-triazolo[1,5-c]quinazoline-5-one-2-carboxylique

6. Acide 7,8-Benzo-7,8,9,10-tétrahydro-1,2,4-triazolo[1,5-c]quinazoline-5-one-2-propionique

7. 8,9-Tétraméthylène-7,8,9,10-tétrahydro-1,2,4-triazolo[1,5-c]quinazoline-5-one-2-carboxylate d'éthyle
